(19) Europäisches Patentamt

European Patent Office

Office européen des brevets

(11) **EP 1 658 809 A1**

(12) **EUROPEAN PATENT APPLICATION**
published in accordance with Art. 158(3) EPC

(43) Date of publication:
**24.05.2006 Bulletin 2006/21**

(51) Int Cl.:
*A61B 5/04* (1968.09)   *A61B 5/05* (1968.09)
*G01N 27/00* (1968.09)   *G01R 29/12* (1968.09)

(21) Application number: **04771608.9**

(22) Date of filing: **06.08.2004**

(86) International application number:
**PCT/JP2004/011633**

(87) International publication number:
**WO 2005/020811 (10.03.2005 Gazette 2005/10)**

(84) Designated Contracting States:
**DE FR GB**

(30) Priority: **29.08.2003 JP 2003308153**

(71) Applicant: **SONY CORPORATION**
**Tokyo 141-0001 (JP)**

(72) Inventor: **Takiguchi, Kiyoaki**
**Tokyo 141-0001 (JP)**

(74) Representative: **Leppard, Andrew John**
**D Young & Co**
**120 Holborn**
**London EC1N 2DY (GB)**

(54) **MEASURING DEVICE AND MEASURING METHOD**

(57)     An object of the present invention is to make it possible to accurately grasp the state inside an object to be measured. According to the present invention there is provided a measuring apparatus comprising: quasi-electrostatic field generating means generating a quasi-electrostatic field of higher strength as compared with a radiated electric field and an induced electromagnetic field; quasi-electrostatic field detecting means detecting a result of interaction between the quasi-electrostatic field generated by the quasi-electrostatic field generating means and applied to a human body, and an electric field corresponding to a potential change caused by a biological reaction inside the human body; and extracting means extracting the potential change from the result of interaction.

Fig. 8

**Description**

Technical Field

The present invention relates to a measuring apparatus and its method which are for example suitably applied to the case where an inner condition of an object to be measured is noninvasively measured.

Background Art

Conventionally, in the case where a human body is measured as an object to be measured, as measuring methods for noninvasively measuring an inner condition of the human body, there have been proposed, for example, X-ray radioscopy, X-ray computed tomography (CT), magnetic resonance imaging (MRI), ultrasonic echo method, Doppler method (see for example Patent Document 1), dielectric spectroscopy (see for example Patent Document 2), near infrared spectroscopy (NIRS) (see for example Non-Patent Document 1) and the like.

Patent Document 1: Japanese Patent Publication No. 6-53117

Patent Document 2: Japanese Patent No. 3367279

Non-Patent Document 1: "Evaluation on Intermittent Claudication using Near Infrared Spectroscopy", H. Tsuchida, et al., Japanese Journal of Vascular Surgery, 1998, VoL.7, No.3, pp.475 to 487

However, the X-ray radioscopy and the X-ray CT, in which radiation rays are used, have a problem of a non-negligible extent of radiation exposure as well as a problem due to temporal and environmental restrictions. Further, in the X-ray CT, in the case of measuring a bloodstream and the like, it is necessary to separately inject a contrast agent and the like. As a result, the bloodstream distribution can be recognized by the contrast agent, but for example the action potential of a nerve (hereinafter referred to as nerve action potential) itself cannot be measured. The nerve action potential is a transient potential change (about +20 (mV)) caused in the inside and the outside of the membrane of a neurone serving as a basic cell of the nerve system when the neurone is stimulated. The nerve action potential is transmitted without attenuation along a nerve axon to its end, and further serves as a stimulus to a subsequent neurone via a synapse (this flow of the nerve action potential is hereinafter referred to as nerve flow).

In the MRI, the distribution of water molecules in a living-body tissue is statically measured by utilizing the nuclear magnetic resonance of water in the living-body tissue. Thus, in order to measure electric phenomena such as the nerve action potential and the nerve flow, or a blood flow in the tissue, some kinds of algorithm to derive the electric phenomena, the blood flow and the like on the basis of the distribution of the water molecules, are required in the MRI, which causes a difficulty.

The ultrasonic echo method, in which the resolution is low and the reflection is caused on the surface of the tissue, is not suitable for a uniform tomographic operation reaching the deep part of the tissue. In addition, in the ultrasonic echo method, for example when the uterus is photographed, clear tomograms cannot be obtained without the urine being stored in the urinary bladder, because of adverse effects of the bladder wall and the like, as a result of which a prescribed restriction of storing the urine in the urinary bladder is forced on a person to be measured. Further, the nerve action potential itself cannot be measured by the ultrasonic echo method either.

In the dielectric spectroscopy, the tissue can be identified better than in the MRI on the basis of the bonded states of water molecules (the states of free water, quasi-bonded water, bonded water) in the tissue. However, in the dielectric spectroscopy, it is difficult to continuously measure a bloodstream and the like for a long period of time. In addition, the dielectric spectroscopy is complicated because it is necessary to perform control of the electrical length and to fix electrodes to the surface of a human body so as to prevent an air gap and a positional deviation from being caused. Further, the nerve action potential itself cannot be measured by the dielectric spectroscopy either.

The Doppler method, in which the Doppler shift due to a bloodstream is measured by irradiating a blood vessel with for example laser light, is a method for individually measuring the bloodstream at a pinpoint of the blood vessel. Therefore, in this method, it is difficult to obtain the distribution of the bloodstream and the blood vessel over a large area. Further, the nerve action potential itself cannot be measured by the Doppler method either.

The near infrared spectroscopy is a method which has been widely recognized in recent years, and in which the fact that light of a specific wavelength in a near-infrared band is hardly absorbed by a living-body tissue and transmitted therethrough, and that the light of the specific wavelength is selectively and specifically absorbed by deoxidized hemoglobin (venous blood) is utilized so as to noninvasively measure the bloodstream distribution of the living-body tissue and the like on the basis of the transmission and the reflection of the light. The near infrared ray has high transmittance, but in practice, is not transmitted in a simple manner as in the case of X-ray due to scattering, refraction and the like in the living body. As a result, in the near infrared spectroscopy, the image in the body tissue except optically shallowly existing or exposed portions such as superficial veins and the retina is difficult to be measured because the near infrared ray is scattered in an extremely complicated manner.

Further, in the near infrared spectroscopy, the main purpose is to measure deoxidized hemoglobin (venous blood), and hence, it is difficult to measure oxidized hemoglobin (arterial blood). That is, the near infrared spectroscopy, in this case, needs complicated estimation algorithm such as for making up in advance a scattering model of a target living-body tissue, and hence, is complicated as well as uneasy in accuracy. Further, the nerve action potential itself cannot be measured by the near infrared spectroscopy either.

On the other hand, there is a method such as magnetoencephalography, which measures a magnetic field caused by the nerve action potential. In this method, when an ion current flows in a living body in accordance with an electrical activity of the living body, such as the nerve action potential, a magnetic field is induced by the ion current as in the case of current flowing through an electric wire, so that the state of the nerve action potential is noninvasively measured by capturing the magnetic field using a highly precise magnetic field sensor. This method is suitable for measuring the two dimensionally distributed nerve action potential of the cerebral neocortex. However, in this method, it is difficult to perform control in the depth direction, such as to three-dimensionally obtain the state under the cortex, so that this method is limited to applications for obtaining the surface activity. Further, in this method, it is difficult to measure a bloodstream simultaneously by the same means. Thus, for this purpose, this method needs to be used in combination with the MRI and the like.

On the other hand, there is known a patch clamp method which is a kind of the voltage-clamp method, as a potential measuring method for the nerve and other cells. The patch clamp method is a method in which a micropipette made of glass is put on a cell membrane under an optical microscope and thereby the open/close state of a targeted ion channel is checked by the channel current. Accordingly, in the patch clamp method, it is necessary not only to control the micropipet under the optical microscope to make the micropipet in contact with the cell membrane, but also to dissect the tissue. For this reason, a noninvasive and non-contact measuring technique is required in this method. Naturally, in the patch clamp method, a bloodstream and the like cannot be measured.

As described above, conventionally, the method for simultaneously measuring different biological reactions such as the bloodstream, the nerve action potential or the like has not yet existed. For this reason, the conventional methods are insufficient for simultaneously obtaining much information on the inner condition of the human body.

Disclosure of the Invention

The present invention has been made in view of the above described circumstances. An object of the present invention is to provide a measuring apparatus and its method which make it possible to more accurately grasp the inner condition of an object to be measured.

In order to solve the above described problems, according to the present invention, there is provided a measuring apparatus comprising:

quasi-electrostatic field generating means generating a quasi-electrostatic field of higher field strength as compared with a radiated electric field and an induced electromagnetic field;
quasi-electrostatic field detecting means detecting a result of interaction between the quasi-electrostatic field generated by the quasi-electrostatic field generating means and applied to an object to be measured and an electric field corresponding to a potential change caused by a dynamic reaction inside the object to be measured; and
extracting means extracting the potential change from the result of interaction detected by the quasi-electrostatic field detecting means.

Further, according to the present invention, there is provided a measuring method comprising: quasi-electrostatic field generating step generating a quasi-electrostatic field of higher field strength of compared with a radiated electric field and an induced electromagnetic field; a quasi-electrostatic field detecting step detecting a result of interaction between the quasi-electrostatic field generated in the quasi-electrostatic field generating step and applied to an object to be measured and an electric field corresponding to a potential change caused by a dynamic reaction inside the object to be measured; and an extracting step to extract the potential change from the result of interaction detected in the quasi-electrostatic field detecting step.

According to the present invention, the measuring apparatus to measure a predetermined object to be measured, comprises:

quasi-electrostatic field generating means generating a quasi-electrostatic field of higher field strength as compared with a radiated electric field and an induced electromagnetic field;
quasi-electrostatic field detecting means detecting a result of interaction between the quasi-electrostatic field generated by the quasi-electrostatic field generating means and applied to an object to be measured and an electric field corresponding to a potential change caused by a dynamic reaction inside the object to be measured; and
extracting means extracting the potential change from the result of interaction detected by the quasi-electrostatic field detecting means,

whereby different dynamic reactions can be simultaneously measured and hence much information inside the object to be measured can be simultaneously obtained.

According to the present invention, the measuring method to measure a predetermined object to be measured, comprises: a quasi-electrostatic field generating step generating a quasi-electrostatic field of higher field strength as compared with a radiated electric field and an induced electromagnetic field; a quasi-electrostatic field detecting step detecting a result of interaction between the quasi-electrostatic field generated in the quasi-electrostatic field generating step and applied to an object to be measured and an electric field corresponding to a potential change caused by a dynamic reaction inside the object to be measured; and an extracting step extracting the potential change from the result of interaction detected in the quasi-electrostatic field detecting step, whereby different dynamic reactions can be simultaneously obtained and hence much information inside the object to be measured can be simultaneously obtained.

Brief Description of the Drawings

Figure 1 is a schematic diagram showing a simulation result (1);

Figure 2 is a schematic diagram showing a simulation result (2);

Figure 3 is a schematic diagram showing a simulation result (3);

Figure 4 is a schematic diagram showing a relative change in each electric field strength (1 (MHz)) with respect to distance;

Figure 5 is a schematic diagram showing a relative change in each electric field strength (10 (MHz)) with respect to distance;

Figure 6 is a schematic diagram showing a quasi-electrostatic field scale (1);

Figure 7 is a schematic diagram showing a quasi-electrostatic field scale (2).

Figure 8 is a schematic block diagram showing a configuration of a measuring apparatus according to the present embodiment;

Figure 9 is a schematic diagram showing a configuration of an electrode for surface measurement;

Figure 10 is a schematic diagram showing an arrangement state of the electrode for surface measurement;

Figure 11 is a flow chart showing a measurement processing procedure;

Figure 12 is a schematic block diagram showing a configuration of a measuring apparatus according to a further embodiment; and

Figure 13 is a schematic diagram showing a state of measurement according to the further embodiment.

Best Mode for Carrying Out the Invention

In the following, the present invention will be described with reference to the accompanying drawings.

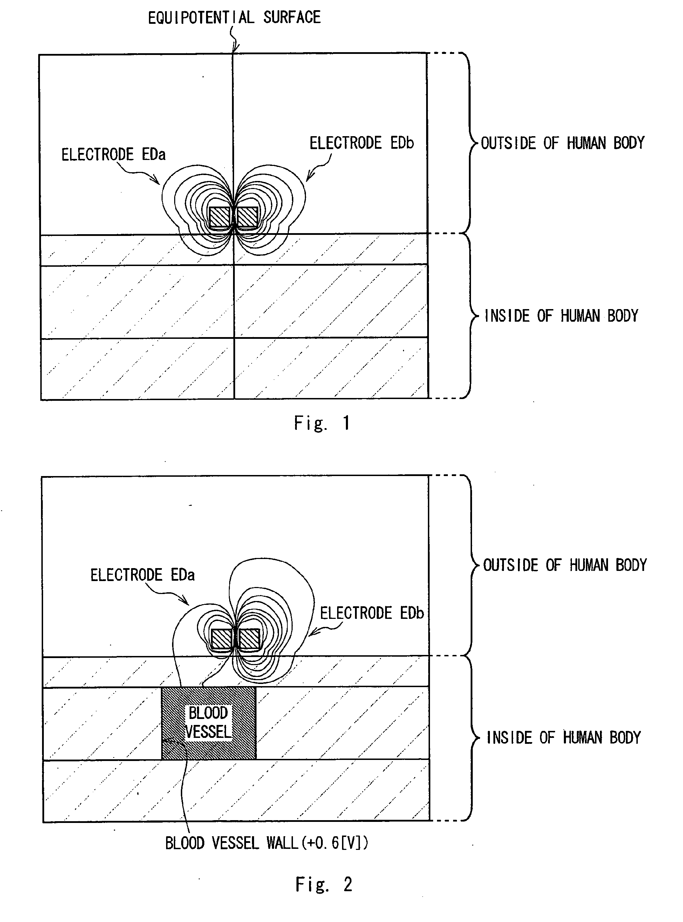

The present invention is directed to measure the inner condition of a human body by utilizing the fact that the human body is an electrostatic conductor as suggested by the empirical fact that the static electricity can be physically experienced in everyday life, that electric fields are formed in accordance with potential changes caused by various biological reactions inside the human body, and that the quasi-electrostatic field has high resolution with respect to distance. First, simulation results of this measuring method are shown in Figure 1 to Figure 3.

(1) Simulation result

Figure 1 to Figure 3 show simulation results in the case where two electrodes EDa and EDb for generating an electric field are arranged in the vicinity of the exterior of a human body, and where a quasi-electrostatic field is generated by applying a voltage to each of the electrodes ED. Noted that in the simulation, the human body is handled by assuming that it has a relative dielectric constant of 50 in a uniform manner.

Figure 1 shows in section a state where no blood vessel exists in the vicinity of the quasi-electrostatic field generated by each of the electrodes ED when the voltage applied to the electrode EDa is 1 (V) and the voltage applied to the electrode EDa is -1 (V). In Figure 1, it can be seen that the equipotential surface whose potential is 0 (V) is generated in the middle between the electrode EDa and the electrode EDb, and that the pattern of the electric field generated by each of the electrodes ED is equivalent to the other.

On the other hand, Figure 2 shows in section a state where a blood vessel exists in the vicinity of the quasi-electrostatic field generated by each of the electrodes ED when a voltage applied to the electrode EDa is 1 (V) and a voltage applied to the electrode EDb is -1(V). However, it is assumed that the blood vessel performs pulsation at 1 to 2 (Hz) and a potential of 0.6 (V) (referred to as electric double layer boundary potential) is formed at an interface between

4

the blood vessel wall and the blood in accordance with the pulsation. In Figure 2, it can be seen that the equipotential surface changes to the side of the electrode EDb as compared with the simulation result in Figure 1 and the pattern of electric field is also correspondingly changed. This means that a positive potential (electric field) exists inside the human body in the vicinity of each of the electrodes ED, and that this change is a result of interaction between the positive potential and the quasi-electrostatic field generated by each of the electrodes ED.

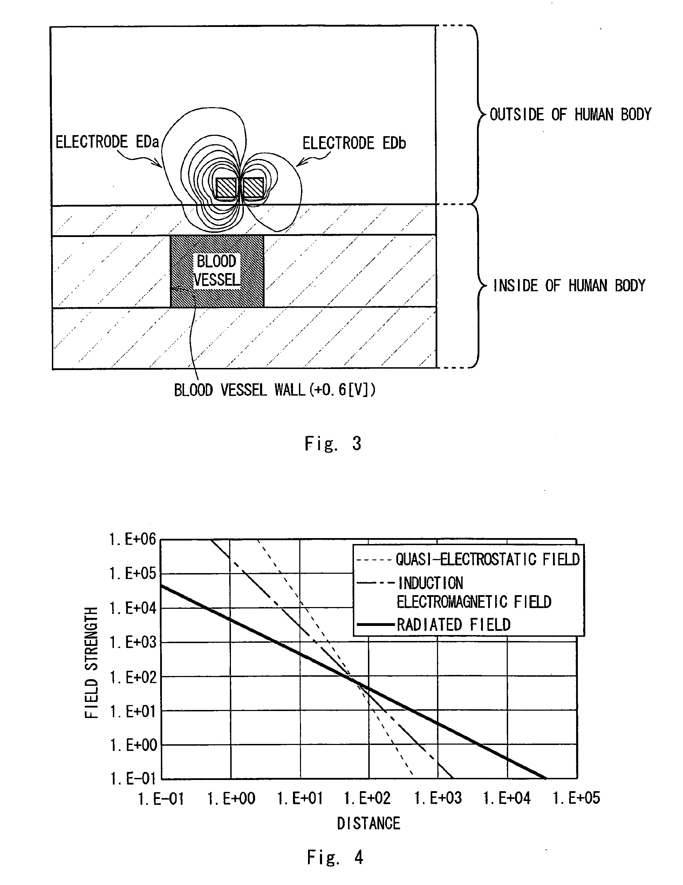

Figure 3 also shows in section a state at the time when the voltages applied to each of the electrodes ED in Figure 2 are reversed (that is, the voltage applied to the electrode EDa is set to -1 (V) and the voltage applied to the electrode EDb is set to 1 (V)). In Figure 3, it can also be seen that the equipotential surface is formed closer to the electrode EDb, similarly to the simulation result shown in Figure 2. This means that the equipotential surface is formed as a result of the interaction between the positive potential (electric field) existing inside the human body in the vicinity of each of the electrodes ED and the quasi-electrostatic fields generated by each of the electrodes ED, similarly to the simulation result shown in Figure 2.

It is seen from the simulation results shown in Figure 1 to Figure 3, that if quasi-electrostatic field detecting means to detect the above described interaction result is provided in the vicinity of the electrodes ED, the potential change caused by a biological reaction can be detected in non-contacting manner from the detection result of the quasi-electrostatic field detecting means.

The strength of the quasi-electrostatic field is inversely proportional to the third power of distance from the electric field sources (electrodes EDa and EDb). This means that the quasi-electrostatic field has a high resolution with respect to the distance. If quasi-electrostatic field generating means to generate a plurality of quasi-electrostatic fields (hereinafter referred to as quasi-electrostatic field scales), each of which has a different reaching distance to the inside of the human body, that is, a different depth range for detecting the effect caused by the biological reaction inside the human body (hereinafter referred to as biological reaction detecting area), is provided by utilizing the property of the quasi-electrostatic field, it becomes possible to measure in layers the inner condition of the human body. Here, before explaining the quasi-electrostatic field scale, the property of the quasi-electrostatic field is explained first.

(2) Property of quasi-electrostatic field

An electric field is generated as a combined electric field of a radiated electric field which is linearly inversely proportional to the distance from the source, an induced electromagnetic field which is inversely proportional to the square of the distance from the source and a quasi-electrostatic field which is inversely proportional to the third power of the distance from the source.

Figure 4 shows a result obtained by graphically illustrating the relationship of the relative strength of each of the radiated electric field, the induced electromagnetic field, the quasi-electrostatic field with respect to the distance. However, in Figure 4, the relationship between the relative strength of each electric field at 1 (MHz) and the distance is shown in a logarithmic scale.

As can be seen from Figure 4, there exists a distance (hereinafter referred to as field strength boundary point) at which the relative strengths of the radiated electric field, the induced electromagnetic field and the quasi-electrostatic field are equal to each other. In this case, at a position far away from the field strength boundary point, the radiated electric field is dominant (in the state where the strength of the radiated electric field is higher than those of the induced electromagnetic field and the quasi-electrostatic field), while at a position nearer than the field strength boundary point, the quasi-electrostatic field is dominant (in the state where the strength of the quasi-electrostatic field is higher than those of the radiated electric field and the induced electromagnetic field).

In deriving the field strength from a maxwell equation, the field strength boundary point can be expressed by the following formula, where r (m) is the distance and k (1/m) is the wave number.

$$r = \frac{1}{k} \qquad \cdots\cdots (1)$$

Then, the wave number k in formula (1) can be expressed by the following formula, where v (m/s) is the propagation velocity of the electric field in a medium and f (Hz) is the frequency.

$$k = \frac{2\pi f}{v} \qquad \cdots\cdots (2)$$

The propagation velocity v of the electric field is expressed by the following formula, where c (m/s) is the light velocity (c = 3 x 10$^8$), and $\varepsilon$ is the relative dielectric constant of the medium.

$$v = \frac{c}{\sqrt{\varepsilon}} \qquad \cdots\cdots (3)$$

Thus, the field strength boundary point can be expressed by the following formula obtained by substituting formula (2) and formula (3) into formula (1) and by arranging the formula resulting from the substitution.

$$r = \frac{c}{2\pi f \cdot \sqrt{\varepsilon}} \qquad \cdots\cdots (4)$$

As can be seen from formula (4), in the case where the space of the quasi-electrostatic field whose strength is higher than those of the radiated electric field and the induced electromagnetic field is increased, the frequency is closely related, and hence in a lower frequency, the space of the quasi-electrostatic field whose strength is higher than those of the radiated electric field and the induced electromagnetic field becomes larger (that is, the distance up to the field strength boundary point shown in Figure 4 becomes longer as the frequency becomes lower (that is, the position is shifted to the right)). On the other hand, in a higher frequency, the space of the quasi-electrostatic field whose strength is higher than those of the radiated electric field and the induced electromagnetic field becomes smaller (that is, the distance up to the field strength boundary point shown in Figure 4 becomes shorter as the frequency becomes higher (that is, the position is shifted to the left)).

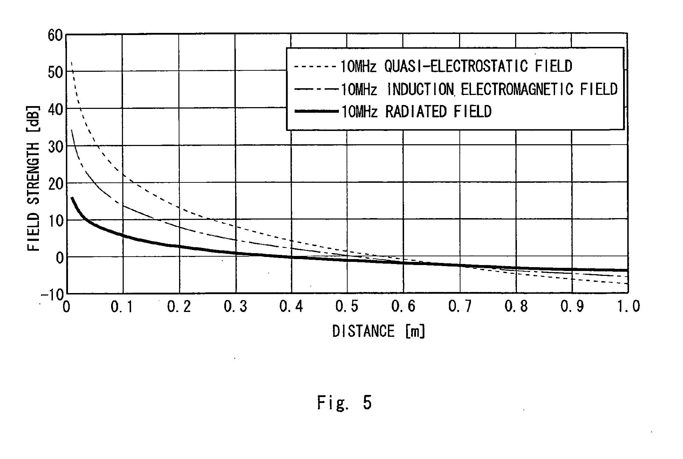

For example, when the frequency of 10 (MHz) is selected, assuming that the relative dielectric constant of a human body is 50 in a uniform manner, the quasi-electrostatic field is dominant in a position nearer than 0.675 (m) from the above described formula (4). Figure 5 shows a result obtained by graphically illustrating the relationship of the relative strength of each of the radiated electric field, the induced electromagnetic field, the quasi-electrostatic field with respect to the distance in the case where the frequency of 10 (MHz) is selected.

As can be seen from Figure 5, in the case where the greatest biological reaction detection area from the electric field source (electrodes EDa and EDb) (the depth range for detecting the effect of the biological reaction inside a human body) is set for example to 0.01 (m), the strength of the quasi-electrostatic field in a position between the electric field source and the position of 0.01 (m) is larger than the strength of the induced electromagnetic field by about 18.2 (dB). Therefore, it can be considered that the quasi-electrostatic field in this case is not affected by the induced electromagnetic field and the radiated electric field.

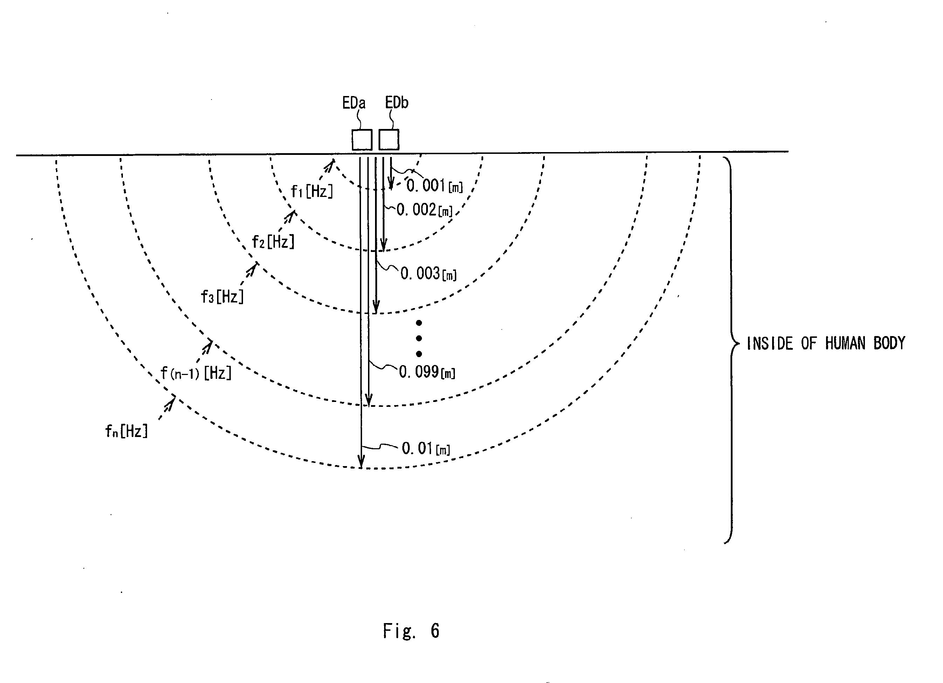

Here, utilizing the above described property of the quasi-electrostatic field, there is described a method for generating a quasi-electrostatic field scale to detect the effect of the biological reaction in the region from the surface of a human body up to a position 0.01 (m) inside the surface of the human body with an interval of 0.001 (m), when the detection is performed from the surface of the human body up to the position of 0.01 (m) inside the surface, for example as shown in Figure 6.

(3) Quasi-electrostatic field scale

As shown in Figure 6, a reference frequency of 10 (MHz) is assigned to the depth of 0.001 (m) which corresponds to a minimum biological reaction detecting area from the surface of the human body, and each time the biological reaction detecting area (that is the depth from the surface of the human body) is successively increased by every depth of 0.001 (m), a frequency corresponding to the detecting area is assigned. In this manner, the biological reaction detecting area of the quasi-electrostatic field can be controlled so as to correspond to the depth of the object to be measured by using the frequencies.

However, in this case, the space in which the quasi-electrostatic field is dominant becomes smaller as the frequency becomes higher, (that is, the field strength boundary point shown in Figure 4 is shifted to the left), so that the difference between the field strengths of the quasi-electrostatic field and the induced electromagnetic field is smaller than 18.2 (dB) in the vicinity of the end of the biological reaction detecting area corresponding to the high frequency. As a result, the field strength of the quasi-electrostatic field scale serving as an indicator for measuring the effect of the biological reaction becomes unstable, and thereby the reliability of measuring accuracy is impaired.

In this case, if the outputs are adjusted so that the field strength at the field strength boundary point corresponding to each frequency $f_{(r)}$ higher than 10 (MHz) matches with the field strength of the biological reaction detecting area (at the depth of 0.001 (m) from the electrode) corresponding to the frequency of 10 (MHz), it is possible to secure the reliability of measuring accuracy because the quasi-electrostatic field becomes stable.

That is, in the case where a sinusoidal wave voltage is outputted to a pair of electrodes for electric field generation to generate a quasi-electrostatic field oscillating in accordance with the frequency of the sinusoidal wave voltage from the electrodes, assuming that a coefficient for performing the above described output adjustment (hereinafter referred to as output adjustment coefficient) is $A_{(r)}$, the field strength $E_{(r)}$ of the quasi-electrostatic field in a biological reaction detecting area (distance) r (m) from the pair of electrodes is expressed by the following formula.

$$E_{(r)} = \frac{A_{(r)}}{r^3} \qquad \cdots\cdots (5)$$

When the biological reaction detecting area (distance) r in formula (5) is modified in accordance with the above described formula (4) relating to the field strength boundary point, the following formula can be obtained.

$$E_{(r)} = \frac{A_{(r)} \cdot c}{\left( 2\pi f \cdot \sqrt{\varepsilon} \right)^3} \qquad \cdots\cdots (6)$$

The frequency $f_{(r)}$ may be determined so that the field strength of the field strength boundary point corresponding to each frequency $f_{(r)}$ higher than 10 (MHz) matches with the field strength in the biological reaction detecting area (at 0.001 (m) from the electrode) corresponding to the frequency of 10 (MHz). Thus, the following formula is established.

$$A_{0.001} = \frac{1}{\left[ \dfrac{c}{2\pi \cdot 10 \times 10^6 \cdot \sqrt{\varepsilon}} \right]^3}$$

$$= \frac{1}{\left[ \dfrac{c}{2\pi f_r \cdot \sqrt{\varepsilon}} \right]^3} \qquad \cdots\cdots (7)$$

By arranging the formula (7), the following formula is obtained.

$$A_r = \left[ \frac{10 \times 10^6}{f_r} \right]^3 A_{0.001} \qquad \cdots\cdots (8)$$

Using the formula (8), it is possible to determine the output coefficient $A_{(r)}$ at the time of outputting the sinusoidal wave voltage of frequency $f_{(r)}$ corresponding to the biological reaction detecting area (distance) r.

Further, the frequency $f_{(r)}$ corresponding to each biological reaction detecting area (distance) r for each depth of 0.001 (m) successively provided from the electrodes for generating the quasi-electrostatic field can be expressed by the following formula.

$$A_{0.001} \frac{1}{0.001^3} = A_r \frac{1}{r^3} \qquad \cdots\cdots (9)$$

By modifying the output coefficient $A_{(r)}$ in formula (9) in accordance with the above described formula (8), the following formula is obtained.

$$A_{0.001} \frac{1}{0.001^3} = \left[\frac{10 \times 10^6}{f^r}\right]^3 A_{0.001} \frac{1}{r^3} \qquad \cdots\cdots (10)$$

Then, the output coefficient $A_{(r)}$ can be determined by using the following formula which is obtained by arranging formula (10).

$$f_r \frac{0.01}{r} \cdot 10 \times 10^6 = \frac{10 \times 10^3}{r} \qquad \cdots\cdots (11)$$

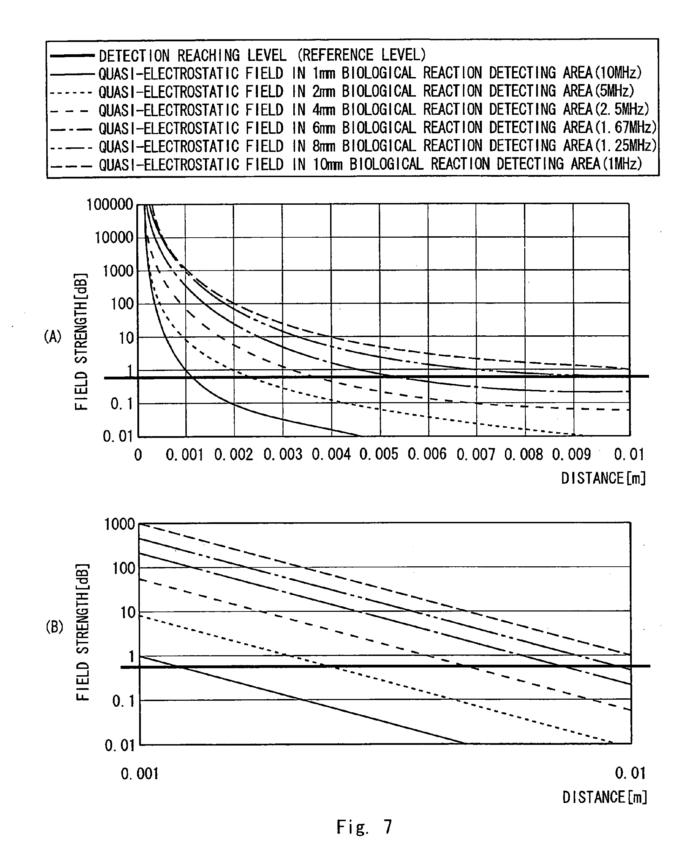

Figure 7 shows a result obtained by graphically illustrating the quasi-electrostatic field scale generated on the basis of each of the above described conditions determined in this manner. However, in Figure 7 for reasons of clarity, the biological reaction detecting areas (distances) for each depth of 0.001 (m) are not shown, but the quasi-electrostatic fields corresponding to only predetermined biological reaction detecting areas (at 0.001 (m), 0.002 (m), 0.004 (m), 0.006 (m), 0.008 (m), 0.01 (m)) are shown. In addition, the vertical axis (field strength) in Figure 7(A), and the vertical axis (field strength) and the horizontal axis (distance) in Figure 7 (B) are shown in a logarithmic scale. As can be seen from Figure 7, when the field strength of a quasi-electrostatic field is fixed for example to the value at the field strength boundary point as a predetermined reference value, the biological reaction detecting area (distance) of the quasi-electrostatic field can be accurately controlled by means of the frequency, so as to correspond to the depth of the object to be measured.

Noted that the case (Figure 6) where the quasi-electrostatic field is generated for each depth of 0.001 (m) from the electrodes for electric field generation is described, but in practice, the size of each depth in which the quasi-electrostatic field is generated is selected in consideration of the distance of the effect of the biological reaction to be detected from the surface of the human body. In this case, after formula (8) and formula (11) are derived on the basis of the selection result, the output adjustment factor and the frequency for generating the quasi-electrostatic field scale having reliability are determined, respectively.

In this manner, the quasi-electrostatic field generating means is capable of generating the quasi-electrostatic field scale having reliability as an indicator for measuring the effect of the biological reaction.

In addition, if the quasi-electrostatic field detecting means is made to detect the result of interaction with the potential change due to the biological reaction within the biological reaction detecting area (distance) corresponding to each frequency of the quasi-electrostatic field scale, the potential change due to the biological reaction inside the human body can be measured in layers.

(4) Configuration of measuring apparatus

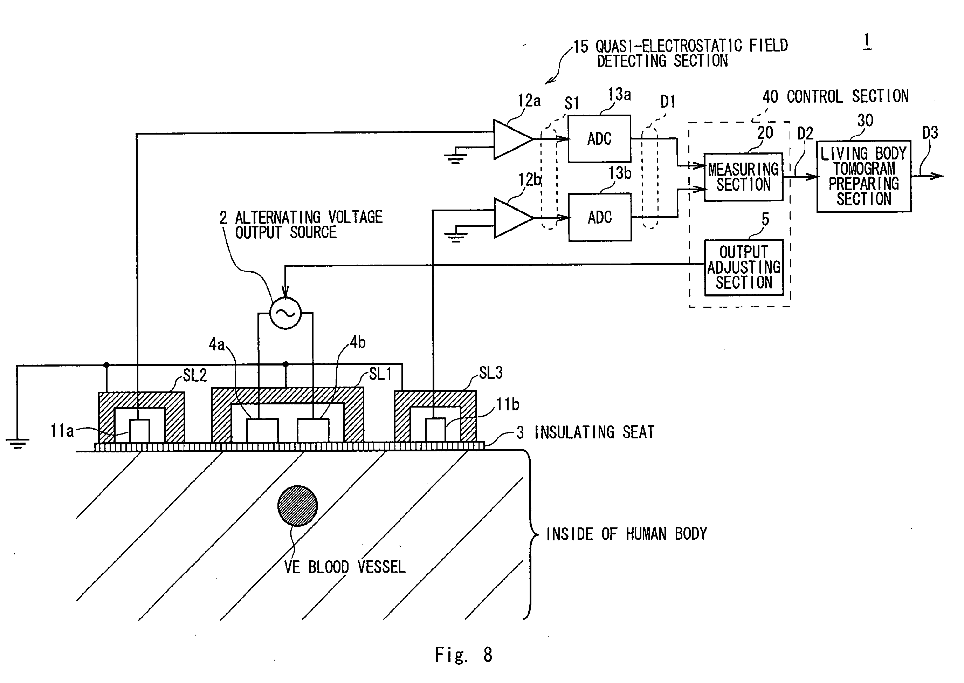

Figure 8 shows a measuring apparatus 1 according to the present embodiment, having quasi-electrostatic field generating means and quasi-electrostatic field detecting means, as described above. That is, in the measuring apparatus 1, the quasi-electrostatic field generating means comprises: an output source 2 (hereinafter referred to as alternating voltage output source) outputting a plurality of sinusoidal wave voltages (hereinafter referred to as alternating voltages) respectively corresponding to a plurality of frequencies; a pair of electrodes for electric field generation 4a and 4b which are connected to the alternating voltage output source 2, and which are arranged at a predetermined position on the surface of a human body via a thin insulating sheet 3 whose dielectric constant is selected so as to be close to that of the air; and an output adjusting section 5 controlling the output of the alternating voltage output source 2.

Each of the sinusoidal wave voltages of alternating voltage in the alternating voltage output source 2 is selected to correspond to each of the frequencies determined in accordance with the above described formula (8). Further, the output adjusting section 5 is arranged to output each of the sinusoidal wave voltages of alternating voltage for every unit time, successively from the sinusoidal wave voltage with lower frequency. At this time, each sinusoidal wave voltage is correspondingly adjusted in accordance with the output adjusting coefficient determined by the above described formula (11) and thereafter outputted to the electrodes for electric field generation 4a and 4b.

As a result, from the electrodes for electric field generation 4a and 4b, the quasi-electrostatic field scales having reliability are successively generated in the time division manner from a quasi-electrostatic field scale having a smaller biological reaction detecting area (distance). In this case, the quasi-electrostatic field with a frequency corresponding to the biological reaction detecting area including a blood vessel VE is changed by the effect of the potential change (electric double layer boundary potential) caused by the biological reaction of the blood vessel VE. At the same time, the quasi-electrostatic fields of each frequency corresponding to the biological reaction detecting areas including various cells (not shown) inside the human body are changed by the effect of the potential charge caused by the biological reactions (for example, neurone stimulation in a nerve cell, and electron transport system in predetermined cells) in the various cell levels in the inside of the human body, respectively.

On the other hand, in the measuring apparatus 1, quasi-electrostatic field detecting means is constituted by a quasi-electrostatic field detecting section 15 which detects a change of the quasi-electrostatic field of the frequency corresponding to each biological reaction detecting area successively generated by the electrodes for electric field generation 4a and 4b, as a signal S1 (hereinafter referred to as field strength change signal) via electrodes for electric field detection 11a, 11b, and amplifiers 12a, 12b. Also, analog digital converters (ADCs) 13a, 13b convert the field strength change signal S1 to detection data D1 (hereinafter referred to as field strength change data), and send the detection data to a measuring section 20.

In this case, the measuring section 20 performs measurement so as to extract a potential change larger than a predetermined set level out of potential changes caused by the biological reaction for each biological reaction detecting area corresponding to each frequency, by applying FFT processing to the field strength change data D1 supplied from the ADCs 13, and sends the measurement result as data D2 (hereinafter referred to as tomographic biological reaction data) to a living body tomogram preparing section 30.

The set level is arranged to be set by the user, and is set for example to the potential change of $\pm 5$ (mV) or more. Therefore, a change in the nerve action potential caused by neurone stimulation, a change in the electric double layer boundary potential by the pulsation of a blood vessel, and the like are made as the object to be extracted, and in this case, the tomographic biological reaction data D2 are made to be data in which the potential changes due to minute biological reactions, for example an electron transport system in the predetermined cells and the like, are eliminated.

The living body tomogram preparing section 30, generates data of living body tomogram (hereinafter referred to as living body tomogram data) D3 by performing a living body tomogram preparing processing using for example an algebraic method on the basis of the tomographic biological reaction data D2, and outputs the living body tomogram data to a display device (not shown). As a result, the state of biological reactions caused by a blood vessel, a nerve and the like under the electrodes for electric field generation 4a and 4b, corresponding to the tomographic biological reaction data D2, are displayed.

In this manner, the measuring apparatus 1 is capable of simultaneously noninvasively measuring different biological reactions for each layer inside the human body, and of providing the measurement result as information.

In addition to the above described configuration, the measuring apparatus 1 comprises a conductive shielding section SL1 surrounding the electrodes for electric field generation 4a and 4b in a state electrically separated from the electrodes 4a and 4b, and conductive shielding sections SL2, SL3 surrounding the electrodes for electric field detection 11a, 11b in a state electrically separated from the electrodes 11a, 11b.

As a result, in the measuring apparatus 1, it is possible to avoid as much as possible the state where the external noise other than the field strength change in the quasi-electrostatic field scale (quasi-electrostatic field with a substantially fixed field strength for each distance corresponding to each of the plurality of frequencies) is detected. Thereby, it is possible to accurately measure the potential change of the biological reaction of very trace amount.

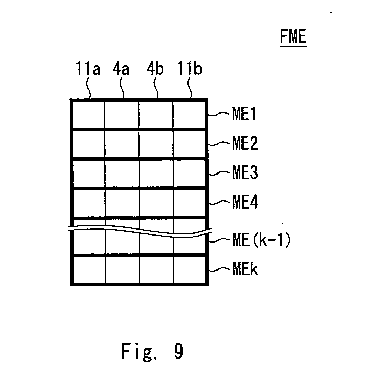

Further, in the measuring apparatus 1 according to the present embodiment, as shown in Figure 9, the electrodes for electric field detection 11a, 11b corresponding to the adjoining electrodes for electric field generation 4a, 4b are linearly arranged so as to be formed as a unit of electrode group ME (hereinafter referred to as unit measuring electrode), and a set of the electrode groups (hereinafter referred to as surface measuring electrode) FME is formed by arranging the unit measuring electrodes in k rows on the same surface.

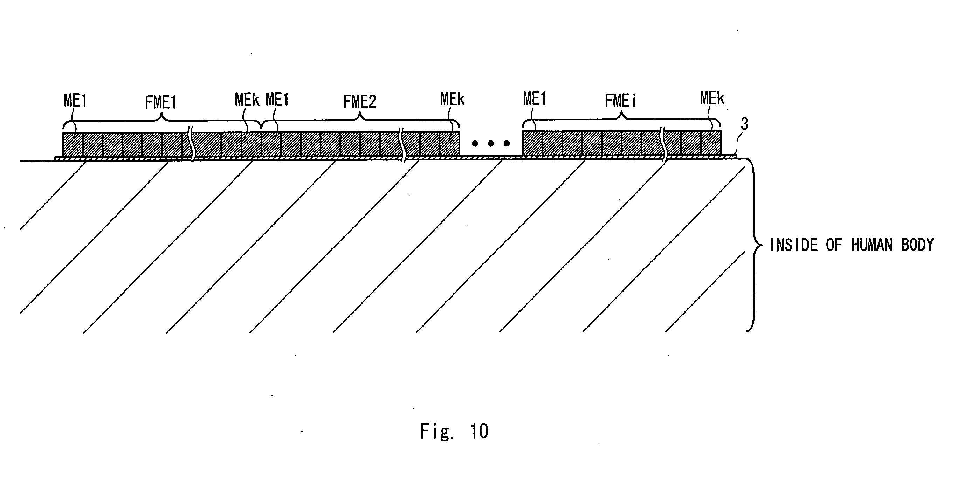

In addition, in the measuring apparatus 1, for example as shown in Figure 10, a plurality of surface measuring electrodes FMEi are provided in mutually adjoining state via the insulating sheet 3.

In this case, the electrodes for electric field generation 4a, 4b (i x k sets of electrodes 4a, 4b) of each unit measuring electrode ME1 to MEk in each surface measuring electrode FMEi are connected to the common alternating voltage output source 2, respectively, while the electrodes for electric field detection 11a, 11b (i x k sets of electrodes 11a, 11b) are connected to commonly corresponding amplifiers 12a, 12b (Figure 8), respectively.

As a result, in the measuring apparatus 1, it is possible to measure different biological reactions for each layer inside the human body over a wider range in real time, whereby for example, a bloodstream and a nerve flow can be dynamically measured so as to be simultaneously followed.

(5) Measurement processing procedure

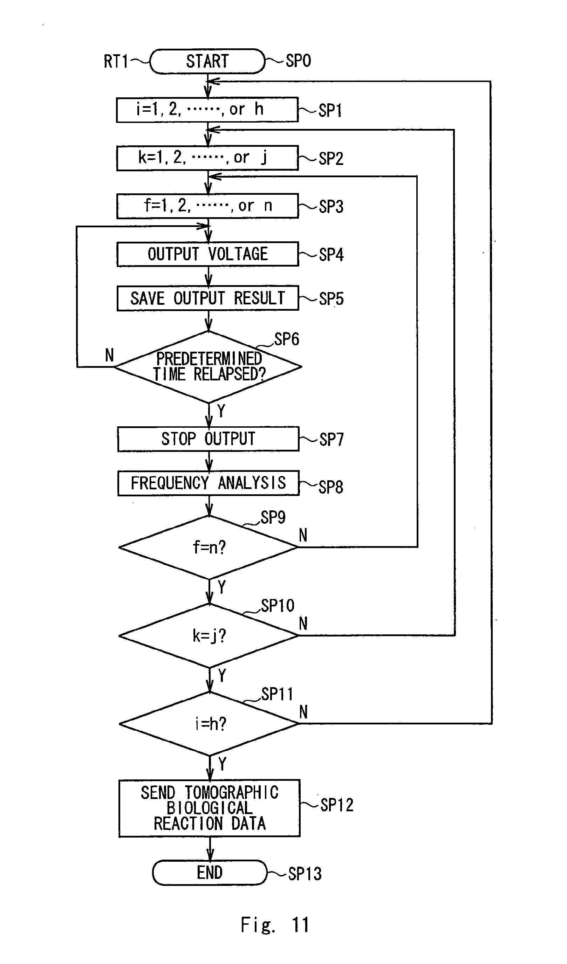

Here, the measurement processing in the control section 40 comprising the output adjusting section 5 and the measuring section 20, is performed in accordance with a measurement processing procedure RT1 shown in Figure 11.

That is, when the main power supply of the measuring apparatus 1 is switched on, the control section 40 starts the measurement processing procedure RT1, and selects in step SP1 the surface measuring electrode FME1 (Figure 10) as the electrode to which a quasi-electrostatic field scale is generated. After selecting the unit measuring electrode ME1 (Figure 9) in step SP2, the control section 40 selects in step SP3 a sinusoidal wave voltage with a minimum frequency f1 (Figure 6) as a frequency to be outputted to the electrodes for electric field generation 4a and 4b of the unit measuring electrode ME1 (Figure 9). In step SP4, the control section 40 outputs the selected sinusoidal wave voltage to the electrodes for electric field generation 4a and 4b.

In this case, the quasi-electrostatic fields (Figure 6) of the biological reaction detecting area up to 0.001 (m) from each of the electrodes for electric field generation 4a and 4b are generated, so that when a blood vessel and the like is present in each layer inside the human body under the electrodes for electric field generation 4a and 4b, the quasi-electrostatic field interacts with the electric field corresponding to the potential change due to the biological reaction of the blood vessel and the like.

Then, in step SP5, the control section 40 temporarily stores in an internal memory the field strength change data D1 (Figure 8) supplied via the corresponding electrodes for electric field detection 11a and 11b as a detection result of the change in the strength of the quasi-electrostatic field in the biological reaction detecting area. In step SP6, the control section 40 judges whether a predetermined period of time has elapsed from the start of the output operation in step SP4, and when an affirmative result is obtained here, stops outputting the sinusoidal wave voltage in step SP7.

Subsequently, the control section 40 applies in step SP8 a frequency analyzing processing to the field strength change data D1 temporarily stored in step SP5 and thereby performs measurement so as to extract the potential change due to the biological reaction in the biological reaction detecting area up to 0.001 (m), which potential change is larger than a set level and temporarily stores the measurement result in the internal memory. Then, in step SP9, the control section 40 judges whether sinusoidal wave voltages of all frequencies fn have been outputted to the electrodes for electric field generation 4a and 4b.

When a negative result is obtained here, this means that the extraction of the potential change due to the biological reaction in all biological reaction detecting areas under the unit measuring electrode ME1 (Figure 9) has not yet completed. At this time, the control section 40 returns to step SP3, and changes the selection of frequency to be outputted from frequency f1 to the subsequent frequency f2, and then repeats the above described processing.

In this manner, when obtaining an affirmative result in step SP9 as a result of repeating the above described processing for sinusoidal wave voltages of all frequencies f1 to fn for the electrodes for electric field generation 4a and 4b of the unit measuring electrode ME1 (Figure 9), the control section 40 judges in step SP10 whether extraction results of the potential change due to the biological reaction in all unit measuring electrodes ME1 to MEk have been obtained.

When a negative result is obtained here, this means that the extraction of the potential change due to the biological reaction in all biological reaction detecting areas under a surface measuring electrode FME1 (Figure 10) has not yet completed. At this time, the control section 40 returns to step SP2, and changes the selection of electrodes to which sinusoidal wave voltages are generated, from the unit measuring electrode ME 1 to the subsequent unit measuring electrode ME2, and then repeats the above described processing.

In this manner, when obtaining an affirmative result in step SP10 as a result of repeating the above described processing for all electrodes of unit measuring electrode ME1 to MEk of the surface measuring electrode FME1 (Figure 10), the control section 40 judges in step SP11 whether extraction results of the potential change due to the biological reaction in all surface measuring electrodes FME1 to FMEi have been obtained.

When a negative result is obtained here, this means that the extraction of the potential change due to the biological reaction in all biological reaction detecting areas under all the surface measuring electrodes FME1 to FMEi (Figure 10) has not yet completed. At this time, the control section 40 returns to step SP1, and changes the selection of electrodes to which sinusoidal wave voltages are generated, from the surface measuring electrode FME1 to the subsequent surface measuring electrode FME2, and then returns to step SP1 and repeats the above described processing.

In this manner, when obtaining an affirmative result in step SP11 as a result of repeating the above described processing operation for all the surface measuring electrodes FME1 to FMEi (Figure 10), the control section 40 generates in step SP12 tomographic biological reaction data D2 on the basis of potential changes due to the biological reaction in all biological reaction detecting areas under all the surface measuring electrodes FME1 to FMEi temporarily stored in step SP8, and sends the generated data to the living body tomogram preparing section 30. After this sending operation, the control section 40 returns to step SP13 and terminates this measurement processing procedure RT1.

In this manner, the control section 40 is arranged to perform the measuring processing.

(6) Operation and effect of present embodiment

In the above described configuration, the measuring apparatus 1 outputs a plurality of sinusoidal wave voltages, each of which has a predetermined frequency, successively from a voltage with lower frequency for every unit time, from the alternating voltage output source 2 to the electrodes for electric field generation 4a and 4b, and thereby generates quasi-electrostatic fields oscillating correspondingly to the frequencies in the time division manner in the state where the field strength of the quasi-electrostatic fields is more dominant than that of the induced electromagnetic field.

Then, the measuring apparatus 1 detects the result of interaction between the quasi-electrostatic fields which are generated by the electrodes for electric field generation 4a and 4b and applied to the human body, and the electric field corresponding to the potential change caused by the biological reaction inside the human body, and performs measurement so as to extract the potential change from the interaction result.

Therefore, in this measuring apparatus 1, it is possible simultaneously detect different biological reactions as potential changes due to the different biological reactions, such as an electric double layer boundary potential of a blood vessel, a nerve action potential and the like, so that much information inside the human body can be simultaneously obtained.

In this case, in the measuring apparatus 1, the outputs of the sinusoidal wave voltage to the electrodes for electric field generation 4a and 4b, each of which outputs corresponds to each of the frequencies, are adjusted so that the strength of each quasi-electrostatic field generated at each distance corresponding to each frequency becomes a predetermined reference field strength.

Therefore, in the measuring apparatus 1, the strength of the quasi-electrostatic field used as an indicator for measuring the effects of the biological reaction can be uniformly generated in the state where the strength of the quasi-electrostatic field is higher than that of the induced electromagnetic field, and hence, it is possible to generate the stable quasi-electrostatic field having reliability in measuring accuracy.

Further, in this case, the measuring apparatus 1 is configured so that a pair of electrodes for generation and a pair of electrodes for detection are formed as a unit electrode, and that the plurality of unit electrodes are formed on a surface. Therefore, in the measuring apparatus 1, the biological reactions which are different for each layer inside the human body, can be measured over a wide range and in real time, as a result of which for example, a bloodstream, a nerve flow and the like can be dynamically measured so as to be followed.

In the above described configuration, the quasi-electrostatic field of higher strength is generated as compared with the radiated electric field and the induced electromagnetic field, and the result of interaction between the quasi-electrostatic field thus generated and applied to the human body, and the electric field caused by the biological reaction inside the human body is detected, so that the measurement is performed so as to extract the potential change from the interaction result. Thereby, different biological reactions can be simultaneously detected and thus much information inside the human body can be simultaneously obtained, as a result of which the inner condition of an object to be measured can be more accurately grasped.

(7) Other embodiments

It is to be noted that in the above described embodiment, there is described the case where the quasi-electrostatic field generating means to generate the quasi-electrostatic field of higher field strength as compared with the radiated electric field and the induced electromagnetic field is constituted by the alternating voltage output source 2, the electrodes for electric field generation 4a and 4b, and the output adjusting section 5, as shown in Figure 8, but the present invention is not limited to the case, and the quasi-electrostatic field generating means may be realized by other various constitutions.

Further, as the generatingmethod of the quasi-electrostatic field generating means for generating the quasi-electrostatic field of higher field strength as compared with the radiated electric field and the induced electromagnetic field, the output adjusting section 5 as output adjusting means is arranged to make the alternating voltage output source 2 output to the electrodes for electric field generation 4a and 4b, sinusoidal wave voltages successively from a voltage with lower frequency in time division manner, thereby generating the quasi-electrostatic fields which makes it possible to obtain the higher field strength as compared with and the induced electromagnetic field, at each distance corresponding to each of the plurality of frequencies in time division manner. However, the present invention is not limited to the case, and the output adjusting section 5 may output to the electrodes for electric field generation 4a and 4b the result of combination of each of the sinusoidal wave voltages, so as to generate the quasi-electrostatic fields which make it possible to obtain the higher field strength as compared with the induced electromagnetic field at each distance corresponding to each of the plurality of frequencies, not in time division manner but simultaneously. In this case, the quasi-electrostatic fields which are the result of combination of the plurality of frequency components are generated simultaneously so that the detected result includes the plurality of frequency components. Thus, it is possible to obtain the same effect as in the above described embodiment by decomposing the detected result for each frequency by the FFT processing.

Further, as the generatingmethodof the quasi-electrostatic field generating means for generating the quasi-electrostatic field of higher field strength as compared with the radiated electric field and the induced electromagnetic field, only a predetermined sinusoidal voltage of the alternating voltage output source 2 may be outputted, whereby the quasi-electrostatic field is selectively generated at a predetermined position inside the human body.

Further, in the above described embodiment, there is described the case where a human body is taken as an object to be measured so that the biological reaction inside the human body is measured. However, the present invention is not limited to the case, and the present invention may be arranged to measure for example the biological reaction inside animals, plants and the like by taking these as objects to be measured, to measure water flows in certain points of ground by taking these as an object to be measured, to measure the biological reaction of survivors present inside collapsed matters collapsed by the disaster and the like by taking these as objects to be measured, to measure a predetermined dynamic response of predetermined fine electronic devices by taking these as objects to be measured, and to measure a predetermined dynamic reaction present in predetermined objects to be conveyed by taking these as objects to be measured, and the like. In addition to these, the present invention may also measure various dynamic reactions inside various objects to be measured.

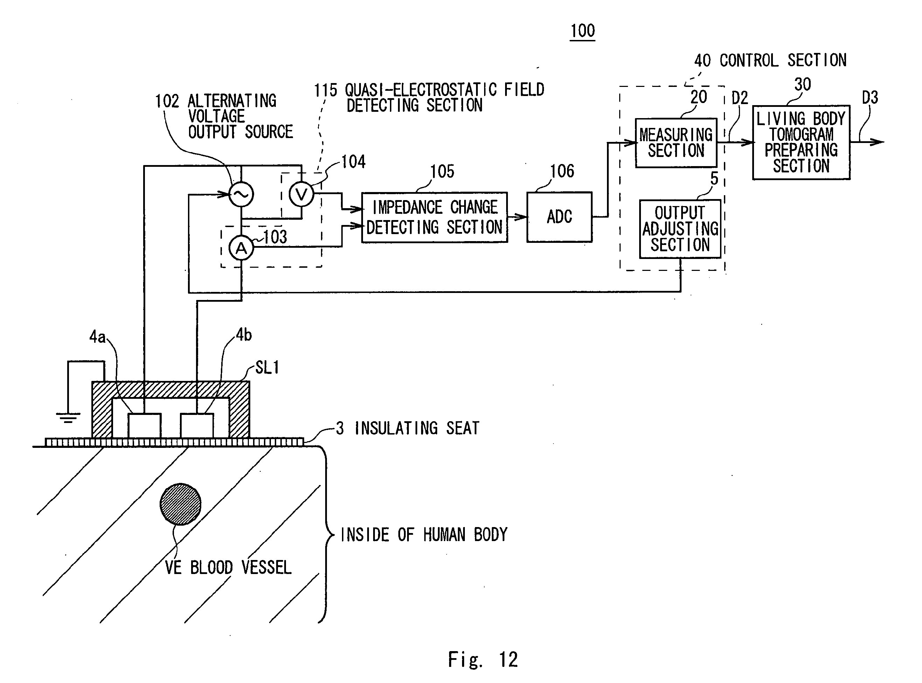

Further, in the above described embodiment, there is described the case where the electrodes for electric field detection 11a, 11b and amplifiers 12a, 12b are arranged as the quasi-electrostatic field detecting means to detect the result of interaction between the quasi-electrostatic field applied to an object to be measured and the electric field corresponding to the potential change caused by the dynamic reaction inside the object to be measured. However, the present invention is not limited to the case. As shown in Figure 12 in which the portions corresponding to those in Figure 8 are denoted by the same reference characters respectively, the interaction result may be detected by an impedance change detecting section 105 which detects an impedance change based on measured values obtained by an ammeter 103 connected between one of the electrodes for electric field generation 4b and the alternating voltage output source 2, and by a voltmeter 104 connected between the outputs of the alternating voltage output source 2, via the ADC 106.

Further, in this case, the interaction result may also be detected by other various kinds of quasi-electrostatic-field detecting means, such as for example, an induction electrode type field strength meter to detect a voltage induced as the induced voltage, an induction electrode modulation amplification type field strength meter in which a DC signal obtained by an induction electrode is converted to AC by using a chopper circuit, an oscillating capacity and the like, an electro-optical effect type field strength meter detecting a change in optical propagation characteristics caused in a material having the electro-optical effect by applying an electric field to the material, an electrometer, a shunt resistance type field strength meter, a current-collecting type field strength meter, and the like.

Further, in the above described embodiment, there is described the case where the measuring section 20 performing FFT processing is applied as the extracting means to extract from the interaction result the potential change caused by the dynamic reaction inside the object to be measured. However, the present invention is not limited to the case, and a measuring section performing frequency analysis processing other than FFT may also be applied.

Further, in the above described embodiment, there is described the case where a unit measuring electrode ME (Figure 9) is formed by linearly arranging the electrodes for electric field detection 11a, 11b corresponding to the electrodes for electric field generation 4a, 4b, and a set of surface measuring electrode FME is formed by arranging the plurality of unit measuring electrodes ME in k rows on the same surface. However, the present invention is not limited to the case, and various unit measuring electrodes ME and surface measuring electrodes FME of which shapes and arrangement states are different from those shown in Figure 9 and Figure 10, may also be formed. What is essential is to form a pair of adjoining electrodes for electric field generation 4a, 4b and a pair of electrodes for electric field detection 11a, 11b adjoining and corresponding to the electrodes 4a, 4b as one unit (a unit measuring electrode ME), and to arrange the plurality of units on the same surface.

Further, in the above described embodiment, there is described the case where the potential change of a blood vessel or a nerve is measured as the potential change due to the biological reaction in the human body. However, the present invention is not limited to the case, and the potential change due to a certain cell itself may be measured.

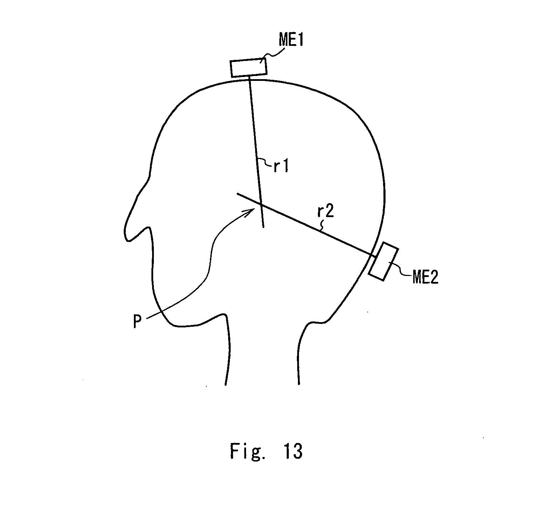

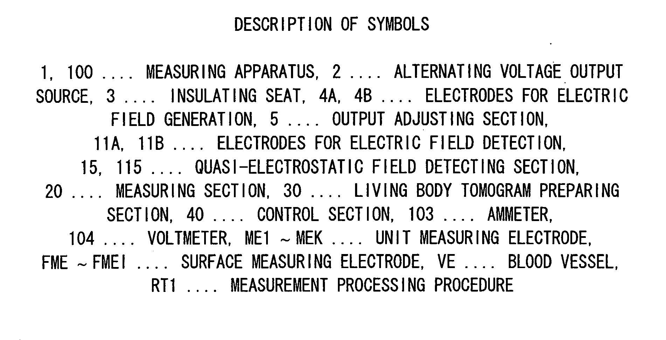

Specifically, as shown in Figure 13, a pair of unit measuring electrodes ME1, ME2, each consisting of electrodes for electric field generation 4a, 4b and electrodes for electric field detection 11a, 11b, each of which electrodes has a size approximately equal to a cell level, are provided so as to make quasi-electrostatic electric fields applied inwardly from the directions different from each other, and the reaching distance (biological reaction detecting areas) of each quasi-electrostatic field applied from both of the unit measuring electrodes ME1, ME2 is successively increased by the output adjusting section 5. At this time, as described above, an intersection point P of the reaching distances r1, r2 of the quasi-electrostatic fields is detected by the impedance change detecting section 105 on the basis of the change in impedances measured by the electrodes for electric field detection 11a, 11b via the ammeter 103 and the voltmeter 104.

At this time, the reaching distances (biological reaction detecting areas) of each of the quasi-electrostatic fields applied from both of the unit measuring electrodes ME1, ME2 are fixed, and the potential change due to the biological reaction in the cell at the intersection point is measured by reversely estimating the impedance change before the intersection point is detected, from the impedance change when the intersection point is detected. In this manner, since the potential change of the biological reaction of a specific cell level can be measured, it is possible not only to avoid that as in the conventional patch clamp method, a micropipet is made to be in contact with a cell membrane and the control of the micropipet is performed under an optical microscope, but also to perform noninvasive and non-contacting measurement.

Further, in the above described embodiment, there is described the case where the quasi-electrostatic fields are generated from the electrodes for electric field generation 4a and 4b. However, in addition to that, according to the present invention, directivity limiting means to limit the directivity of the quasi-electrostatic fields for example in a linear state may also be provided for the electrodes for electric field generation 4a, 4b. Thereby, it is possible to perform a detecting operation specialized for the result of interaction with the dynamic reaction inside the object to be measured without detecting the result of interaction with external noises, as a result of which the measuring accuracy can be further enhanced.

Further, in the above described embodiment, there is described the case where the quasi-electrostatic field is taken as the object to be measured in order to measure the potential change due to the biological reaction inside the human body. However, the present invention is not limited to the case, and a quasi-electrostatic field for treatment may also be generated as the object to be measured simultaneously with the measurement. In this case, it is possible not only to perform the treatment in non-contacting manner but also to measure the effect of the treatment in real time, and to thereby effect simplification at the time of surgery and study.

Further, in the above described embodiment, there is described the case where the change of the electric double layer boundary potential caused by the pulsation of a blood vessel is arranged to be measured. However, according to the present invention, in addition to that, it is also possible to measure the pulsation itself by taking a time-base area into account.

Further, in the above described embodiment, there is described the case where the living body tomogram preparing section 30 which generates living body tomogram data D3 on the basis of a measurement result (tomographic biological reaction data D2) and outputs the tomogram data to the display section (not shown), is arranged to be provided. However, the present invention is not limited to the case, and a discriminating section to discriminate an acute lesion and other diseases on the basis of the measurement result may also be provided. By this manner, a simple diagnosis can be performed simultaneously with the measurement.

Further, in the above described embodiment, there is described the case where the living body tomogram preparing section 30 which generates living body tomogram data D3 on the basis of a measurement result (tomographic biological reaction data D2), and outputs the tomogram data to the display section (not shown), is arranged to be provided. However, instead of this, according to the present invention, an authentication information generating section which generates authentication information used in performing a predetermined authentication processing and outputs the authentication information to an external apparatus, may also be provided. Thereby, it is possible to use the biological reaction formed in a pattern specific to a human body as the authentication information so that the confidentiality of information in the external apparatus can be further secured.

Further, in the above described embodiment, there is described the case where the result of the interaction between the quasi-electrostatic field generated by the quasi-electrostatic field generating means and applied to a human body and the electric field corresponding to the potential change caused by the biological reaction inside the human body is arranged to be detected, and where the potential change is extracted on the basis of the detection result. However, the present invention is not limited to the case, and for example, as in an example that a shark, a ray and the like detect electric fields (quasi-electrostatic fields) generated in living bodies by means of an organ referred to as ampulla of Lorenzini which is present in their head, to thereby identify a living body as a bait for themselves among the living bodies, it may also be arranged that the potential change caused by the biological reaction inside the living body is directly detected by the above described quasi-electrostatic field detecting means, and the potential change caused by a predetermined biological reaction is extracted from the levels of the detected potential change, by referring for example to a table in which the levels of the potential change and the kinds of the biological reaction are made in advance to correspond to each other.

Industrial Applicability

The present invention is applicable in the case of noninvasively measuring inner conditions of an object to be measured, such as a living body, a predetermined electronic device and ground.

**Claims**

1. A measuring apparatus comprising:

   quasi-electrostatic field generating means generating a quasi-electrostatic field of higher field strength as compared with a radiated electric field and an induced electromagnetic field;
   quasi-electrostatic field detecting means detecting a result of interaction between said quasi-electrostatic field generated by said quasi-electrostatic field generating means and applied to an object to be measured, and an electric field corresponding to a potential change caused by a dynamic reaction inside said object to be measured; and
   extracting means extracting said potential change from said result of interaction detected by said quasi-electrostatic field detecting means.

2. The measuring apparatus according to claim 1, wherein:

   said object to be measured is a living body; and
   said quasi-electrostatic field detecting means detects said result of interaction with said electric field corresponding to said potential change caused by a biological reaction inside said living body.

3. The measuring apparatus according to claim 1, wherein
   said quasi-electrostatic field generating means generates said quasi-electrostatic fields of said higher field strength as compared with said induced electromagnetic field, at each of said distances respectively corresponding to said plurality of frequencies.

4. The measuring apparatus according to claim 1, wherein
   said quasi-electrostatic field generating means generates said quasi-electrostatic fields of said higher field strength as compared with said induced electromagnetic field, in time division manner for each of said distances at each of said distances respectively corresponding to said plurality of frequencies.

5. The measuring apparatus according to claim 3, wherein
   said quasi-electrostatic field generating means comprises output adjusting means adjusting outputs of each voltage corresponding to each of said frequencies to a predetermined electrode, to make the strength of each of said quasi-electrostatic fields generated at each of said distances respectively corresponding to each of the frequencies become a predetermined field strength, and outputting a combined result of each of said voltages after the adjustment.

6. The measuring apparatus according to claim 4, wherein
   said quasi-electrostatic field generating means comprises output adjusting means adjusting outputs of each voltage corresponding to each of said frequencies to a predetermined electrode, to make the strength of each of said quasi-electrostatic fields generated at each of said distances respectively corresponding to each of the frequencies become a predetermined field strength.

7. The measuring apparatus according to claim 1, wherein:

   said quasi-electrostatic field generating means comprises a pair of electrodes for generation generating said quasi-electrostatic fields;
   said quasi-electrostatic field detecting means comprises a pair of electrodes for detection detecting said result of interaction; and
   said pair of electrodes for generation and said pair of electrodes for detection are formed into a unit electrode and a plurality of said unit electrodes are formed on the same surface.

8. A measuring method comprising:

   a quasi-electrostatic field generating step generating a quasi-electrostatic field of higher field strength as compared with a radiated electric field and an induced electromagnetic field;
   a quasi-electrostatic field detecting step detecting a result of interaction between said quasi-electrostatic field generated in said quasi-electrostatic field generating step and applied to an object to be measured, and an electric field corresponding to a potential change caused by a dynamic reaction inside said object to be measured; and

an extracting step extracting said potential change from said result of interaction detected in said quasi-electrostatic field detecting step.

9. The measuring method according to claim 8, wherein
said object to be measured is a living body, and wherein said result of interaction with said electric field corresponding to said potential change caused by a biological reaction inside said living body is detected in said quasi-electrostatic field detecting step.

10. The measuring method according to claim 8, wherein
said quasi-electrostatic fields of said higher field strength as compared with said induced electromagnetic field at each of said distances respectively corresponding to a plurality of said frequencies are generated in said quasi-electrostatic field generating step.

11. The measuring method according to claim 8, wherein
said quasi-electrostatic fields of said higher field strength as compared with said induced electromagnetic field are generated in time division manner for each of said distances at each of said distances respectively corresponding to a plurality of said frequencies in said quasi-electrostatic field generating step.

12. The measuring method according to claim 10, wherein
said quasi-electrostatic field generating step comprises output adjusting step adjusting outputs of each voltage corresponding to each of said frequencies to a predetermined electrode, to make the strength of each of said quasi-electrostatic fields generated at said distances respectively corresponding to each of the frequencies become a predetermined field strength, and outputting a combined result of each of said voltages after the adjustment.

13. The measuring method according to claim 11, wherein
said quasi-electrostatic field generating step comprises output adjusting step adjusting outputs of each voltage corresponding to each of said frequencies to a predetermined electrode, to make the strength of each of said quasi-electrostatic fields generated at said distances respectively corresponding to each of the frequencies become a predetermined field strength.

14. A measuring apparatus comprising:

quasi-electrostatic field detecting means detecting potential changes caused by biological reactions inside a living body; and
extracting means extracting one of said potential changes caused by predetermined one of said biological reactions from said potential changes detected by said quasi-electrostatic field detecting means.

15. A measuring method comprising:

quasi-electrostatic field detecting step detecting potential changes caused by biological reactions inside a living body; and
extracting step extracting one of said potential change caused by predetermined one of said biological reactions from said potential changes detected in said quasi-electrostatic field detecting step.

EQUIPOTENTIAL SURFACE

ELECTRODE EDa          ELECTRODE EDb

OUTSIDE OF HUMAN BODY

INSIDE OF HUMAN BODY

Fig. 1

ELECTRODE EDa

ELECTRODE EDb

OUTSIDE OF HUMAN BODY

BLOOD VESSEL

INSIDE OF HUMAN BODY

BLOOD VESSEL WALL (+0.6[V])

Fig. 2

Fig. 3

Fig. 4

Fig. 5

EP 1 658 809 A1

EDa  EDb

f₁[Hz]

0. 001[m]

0. 002[m]

f₂[Hz]

0. 003[m]

f₃[Hz]

INSIDE OF HUMAN BODY

f(n-1)[Hz]

0. 099[m]

fₙ[Hz]

0. 01[m]

Fig. 6

Fig. 7

Fig. 8

1 — QUASI-ELECTROSTATIC FIELD DETECTING SECTION 15

40 CONTROL SECTION

LIVING BODY TOMOGRAM PREPARING SECTION 30

MEASURING SECTION 20

OUTPUT ADJUSTING SECTION 5

ADC 13a · ADC 13b

S1 · 12a · 12b

D1 · D2 · D3

2 ALTERNATING VOLTAGE OUTPUT SOURCE

SL3 · SL1 · SL2

11b · 4b · 4a · 11a

3 INSULATING SEAT

INSIDE OF HUMAN BODY

VE BLOOD VESSEL

FME

11a  4a  4b  11b

ME1
ME2
ME3
ME4
ME(k-1)
MEk

Fig. 9

Fig. 10

RT1 — ( START ) — SP0

i=1, 2, ……, or h — SP1

k=1, 2, ……, or j — SP2

f=1, 2, ……, or n — SP3

OUTPUT VOLTAGE — SP4

SAVE OUTPUT RESULT — SP5

SP6
PREDETERMINED
TIME RELAPSED?
N

Y

STOP OUTPUT — SP7

FREQUENCY ANALYSIS — SP8

SP9
f=n?
N

Y

SP10
k=j?
N

Y

SP11
i=h?
N

Y

SEND TOMOGRAPHIC
BIOLOGICAL
REACTION DATA — SP12

( END ) — SP13

Fig. 11

Fig. 12

Fig. 13

DESCRIPTION OF SYMBOLS

1, 100 .... MEASURING APPARATUS, 2 .... ALTERNATING VOLTAGE OUTPUT
SOURCE, 3 .... INSULATING SEAT, 4A, 4B .... ELECTRODES FOR ELECTRIC
FIELD GENERATION, 5 .... OUTPUT ADJUSTING SECTION,
11A, 11B .... ELECTRODES FOR ELECTRIC FIELD DETECTION,
15, 115 .... QUASI-ELECTROSTATIC FIELD DETECTING SECTION,
20 .... MEASURING SECTION, 30 .... LIVING BODY TOMOGRAM PREPARING
SECTION, 40 .... CONTROL SECTION, 103 .... AMMETER,
104 .... VOLTMETER, ME1 ~ MEK .... UNIT MEASURING ELECTRODE,
FME ~ FMEI .... SURFACE MEASURING ELECTRODE, VE .... BLOOD VESSEL,
RT1 .... MEASUREMENT PROCESSING PROCEDURE

## INTERNATIONAL SEARCH REPORT

| International application No. |
| --- |
| PCT/JP2004/011633 |

| A. CLASSIFICATION OF SUBJECT MATTER |
| --- |
| Int.Cl$^7$ A61B5/04, A61B5/05, G01N27/00, G01R29/12 |

According to International Patent Classification (IPC) or to both national classification and IPC

| B. FIELDS SEARCHED |
| --- |
| Minimum documentation searched (classification system followed by classification symbols)<br>Int.Cl$^7$ A61B5/04, A61B5/05, G01N27/00, G01R29/12 |

Documentation searched other than minimum documentation to the extent that such documents are included in the fields searched
| Jitsuyo Shinan Koho | 1922–1996 | Toroku Jitsuyo Shinan Koho | 1994–2004 |
| --- | --- | --- | --- |
| Kokai Jitsuyo Shinan Koho | 1971–2004 | Jitsuyo Shinan Toroku Koho | 1996–2004 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X<br>A | JP 8-140954 A (NEC Corp.),<br>04 June, 1996 (04.06.96),<br>Par. No. [0001]<br>(Family: none) | 14<br>1-7 |
| X<br>A | JP 55-2416 A (Jun'ichi SUSUKI),<br>09 January, 1980 (09.01.80),<br>Page 1, lower left column, lines 7 to 9;<br>lower right column, lines 15 to 20<br>(Family: none) | 14<br>1-7 |
| X<br>A | JP 2003-232823 A (Atsushi NAKAZOE),<br>22 August, 2003 (22.08.03),<br>Abstract<br>(Family: none) | 14<br>1-7 |

☐ Further documents are listed in the continuation of Box C.　　☐ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search<br>09 November, 2004 (09.11.04) | Date of mailing of the international search report<br>22 November, 2004 (22.11.04) |
| --- | --- |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

| International application No. |
| --- |
| PCT/JP2004/011633 |

---

**Box No. II**     **Observations where certain claims were found unsearchable (Continuation of item 2 of first sheet)**

This international search report has not been established in respect of certain claims under Article 17(2)(a) for the following reasons:

1. ☒ Claims Nos.: 8-13, 15
   because they relate to subject matter not required to be searched by this Authority, namely:
   Claims 8 to 13 and 15 are relevant to diagnostic methods to be practiced on the human body and thus relate to a subject matter which this International Searching Authority is not required, under the provisions of Article 17(2)(a)(i) of the PCT    (continued to extra sheet.)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III**     **Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**       ☐   The additional search fees were accompanied by the applicant's protest.

                            ☐   No protest accompanied the payment of additional search fees.

---

Form PCT/ISA/210 (continuation of first sheet (2)) (January 2004)

**INTERNATIONAL SEARCH REPORT**

International application No.

PCT/JP2004/011633

Continuation of Box No.II-1 of continuation of first sheet(2)

and Rule 39.1(iv) of the Regulations under the PCT, to search.

Form PCT/ISA/210 (extra sheet) (January 2004)